# EUROPEAN PATENT APPLICATION

(11) **EP 3 305 366 A1**
(43) Date of publication of application: **11.04.2018**
(21) Application number: 16192770.2
(22) Date of filing: 07.10.2016
(51) Int. Cl.: A61N 5/10

(54) **HADRON THERAPY APPARATUS FOR ADAPTIVE TREATMENT IN NON-SUPINE POSITION**

(71) Applicant: ION BEAM APPLICATIONS S.A., 1348 Louvain-la-Neuve (BE)
(72) Inventor: FORTON, Eric, 1348 Louvain-la-Neuve (BE); BERTRAND, Damien, 1348 Louvain-la-Neuve (BE); CLAEREBOUDT, Yves, 1348 Louvain-la-Neuve (BE); VAN DER KRAAIJ, Erik, 1348 Louvain-la-Neuve (BE)
(74) Representative: Pecher, Nicolas

(57) **Abstract**

The invention relates to an apparatus comprising a hadron therapy device and a magnetic resonance imaging device (MRI). The MRI is an open MRI, for acquiring magnetic resonance data in the MRI imaging volume. The nozzle is fixed and positioned for directing a beam along a beam path substantially along said axis, or substantially perpendicularly to said axis. The apparatus comprises a patient support system adapted for supporting a patient in a non-supine position in said MRI. The invention also relates to methods for adapting a treatment plan to movements of organs resulting from displacement of a patient from a supine position wherein treatment plan imaging was performed to a non-supine position for performing the treatment.

## Description

### Field of the invention

The invention relates to a medical apparatus comprising a hadron therapy device and a magnetic resonance imaging device, and to a method for preparing a treatment plan for use in such a device.

### Description of prior art

Hadron therapy (for example, proton therapy) for treating a patient has been known for a couple of decades with the prospect of several advantages over conventional radiotherapy. These advantages are due to the physical nature of hadrons, including the fact that energy deposition curve in matter occurs along a curve presenting a sharp peak, the Bragg peak.

In practice, hadron therapy usually requires the establishment of a treatment plan before any treatment can start. During this treatment plan, a 3D image, which may be a computer tomography scan (CT scan), an MRI image, and/or a PET scan, of the patient and target tissues is usually performed. The 3D image is used to characterize the target tissue and the surrounding tissues to be traversed by a treatment hadron beam for the treatment of a patient. The characterization yields a 3D representation of the volume comprising the target tissue, and a treatment plan system determines a range-dose calculated based on the nature of the tissues traversed by the hadron beam. The 3D image is preferably taken with a patient in supine position, i.e. lying horizontally with the face and torso facing up. In this position, the organs take a natural rest position. The treatment is also preferably performed with the patient in the same supine position. This minimizes the risks that organs may have moved between the acquisition of the 3D image and the treatment.

The treatment plan can then be executed during a treatment phase including one or more treatment sessions during which doses of hadrons are deposited onto the target tissue. The position of the Bragg peak of a hadron beam with respect to the target spots of a target tissue, however, suffers of a number of uncertainties including:
- the variations of the patient position, on the one hand, during a hadron therapy session and, on the other hand, between the establishment of the treatment plan and the hadron therapy session;
- the variations of the size and/or of the position of the target tissue and/or of the healthy tissues positioned upstream from the target tissue with respect to the hadron beam (the variation in position of the organs and target tissue may be important when the patient is positioned during treatment in a non-supine position, e.g. seated or standing);

The uncertainty on the position of the patient and, in particular, of the target tissue is critical for obvious reasons. Even with an accurate characterization by the 3D image, the actual position of a target tissue during a treatment session remains difficult to ascertain for the following reasons:
- first, during an irradiation session, the position of a target tissue can change because of anatomical processes such as breathing, digestion, or heartbeats of the patient. Anatomical processes can also cause gases or fluids appearing or disappearing from the path of a hadron beam.
- second, treatment plans are usually determined several days or weeks before a hadron treatment session starts and treatment of a patient can take several weeks distributed over several treatment sessions. During this time period, the patient can lose or gain weight, therefore modifying, sometimes significantly, the volume of tissues such as fats and muscles.

The use of a magnetic resonance imaging device (MRI) coupled to a hadron therapy device has been proposed in the art for identifying any variation of the size and /or the position of a target tissue. For example, US patent 8427148 describes a system comprising a hadron therapy device coupled to a MRI. Said system can acquire images of the patient during a hadron therapy session and can compare these images with CT scan images of the treatment plan. An example of a suitable MRI includes, but is not limited to, a device described in EP0186238.

Known hadron therapy devices may comprise a plurality of treatment rooms. A typical installation such as described in US4870287 comprises three treatment rooms having a gantry and one treatment room having a fixed beam. Gantry treatment rooms allow irradiation of a patient from any direction, by positioning the gantry at a desired angle. This, in combination with a rotation of the patient support, allows full flexibility, i.e. irradiation of the patient from any direction in a full sphere (4π). This flexibility is desired by the therapist establishing the treatment plan, but represents a significant space and cost. The Gantry is a large mechanical structure, supporting heavy magnets for guiding the beam. The structure of a gantry for proton therapy may have a length of 10m, a diameter of 10m and a weight of 100 ton. A gantry for carbon therapy may be much larger and heavier (length 25m, diameter 13m, weight 600 ton). The cost of the gantry, including the shielding enclosing said gantry may represent 60% of the cost of a treatment room.

Therefore, there is an interest for treatment rooms having one or more fixed beam lines. An example of such a hadron therapy device is disclosed in WO03092812. This device comprises a plurality of fixed magnetic channels, in a vertical plane. A deflecting magnet is provided at the end of each channel, for directing the beam from a wider range of directions.

Hadron therapy devices having a fixed beam line, more specifically a horizontal fixed beam line are known e.g. at the Harvard Cyclotron Laboratory. In this centre, treatments of patients have been performed, especially for intercranial and eye tumours. It is convenient to treat such tumours with a fixed horizontal beam line, because the problem discussed above of organs moving inside the patient body do not occur for such tumours. However, there is a need for hadron therapy device having a fixed beam line, allowing to treat any tumours in the body.

### Summary of the invention

It is an object of the present invention to provide a medical apparatus comprising a hadron therapy device having a fixed beam line, adapted for treating a patient in a non-supine position.

The invention is defined by the independent claims. The dependent claims define advantageous embodiments.

In a first aspect, there is provided a medical apparatus comprising:
a) a hadron therapy device comprising a hadron source comprising a nozzle, adapted for directing a hadron treatment beam along a beam path to a target volume;
b) a magnetic resonance imaging device (MRI).

### According to the invention,

• the MRI comprises two coils arranged at a distance and configured for generating a magnetic field in a direction along an axis in an MRI imaging volume between said two coils and around said axis, for acquiring magnetic resonance data in said MRI imaging volume, said MRI imaging volume encompassing said target volume, said imaging volume having a centre;
• said nozzle is fixed and positioned for directing a beam along a beam path substantially along said axis, the angle between said axis and said beam path being smaller or equal to 20°, or substantially perpendicularly to said axis, the angle between a perpendicular to said axis and said beam path being smaller or equal to 20°;
• it comprises a patient support system adapted for supporting a patient in a non-supine position.

In an embodiment, said patient support system is adapted for supporting a patient in a seated position. The seated position may comprise positions with the patient leaning backwards or forwards.

In another embodiment, said patient support system is adapted for supporting a patient in a standing position. The support may have a vertical part with means for immobilizing the patient, the patient having his back or his front in contact to the vertical part.

Said patient support system may be adapted for being rotated around a vertical axis.

In still another embodiment, said patient support system is adapted for supporting a patient in a prone position. The patient may be lying on a generally flat support or a support having a shape adapted to the morphology of the patient, depending on the needs of the treatment. Also, the patient may be bound to a support by immobilization means.

Said patient support system may then be adapted for being rotated around a horizontal axis, said axis being a longitudinal axis of said patient support.

Preferably, the apparatus comprises a controller adapted for instructing said apparatus to perform the method of the invention.

Preferably, said nozzle is positioned at a distance larger than 2m, preferably larger than 3m from said center of said MRI imaging volume. The distance is measured from the point where the hadron beam exits the nozzle to the center of the MRI imaging volume.

In a preferred embodiment, said nozzle is positioned for directing a beam in a first direction along a beam path substantially along said axis and comprises a first pair of magnets S1x, S1y configured for steering the beam in two orthogonal directions both perpendicular to said first direction, and a second pair of steering magnets S2x, S2y configured for steering the beam in a direction parallel to said first direction.

In a second aspect of the invention, there is provided a method for preparing a treatment plan for treating a patient using the apparatus of any of claims 1 to 9 comprising the steps of:
- obtaining a 3D image of a target volume of said patient using one of the following imaging means: a CT-scan, an MRI, a PET imaging system, said patient being in a supine positon;
- determining a treatment plan based on said 3D image;
- determining a displacement of said target volume within said patient resulting from said patient being moved from said supine to said non-supine position;
- adapting said treatment plan to said displacement;
- positioning said patient in said apparatus said patient being in said non-supine position.

Said step of determining a displacement may comprise the steps of:
- obtaining a first MRI image from said MRI, the patient being in said non-supine position;
- obtaining a second MRI image from said MRI, the patient being in a supine position;
- determining said displacement of said target region from the comparison of said first and second MRI image.

Alternatively, said step of determining a displacement may comprise the steps of:
- obtaining a first MRI image from said MRI, the patient being in said non-supine position;
- determining said displacement of said target region from the comparison of said first MRI image and said 3D image.

In a third aspect of the invention, there is provided a method for preparing a treatment plan for treating a patient using the apparatus of any of claims 1 to 9 comprising the steps of:
- obtaining a 3D image of a target region of said patient using one of the following imaging means: a CT-scan, an MRI, a PET imaging system, said patient being in a non-supine positon;
- determining a treatment plan based on said 3D image;
- positioning said patient in said apparatus said patient being in said non-supine position.

In a fourth aspect of the invention, there is provided a method for treating a patient using the apparatus of any of claims 1 to 9 wherein said treatment plan comprises a plurality of spots each having a spot position in said target region, comprising the steps of:
1) performing the method of any of claims 10 to 13;
2) irradiating one or more spots of said plurality of spots
3) acquiring an image of said MRI imaging volume using said MRI;
4) comparing said image with said 3D image and adapting said treatment plan according to the differences;
5) repeating steps 2) to 4) until all spots of said plurality have been irradiated.

In the context of the present invention, a nozzle that is fixed and positioned for directing a beam has to be construed as a nozzle of what is generally named, as discussed above, a fixed beam line, i.e. a nozzle that cannot move. Typically the nozzle of a fixed beam line is attached to a support that is fixed to the floor level of the treatment room, in contrast to a nozzle mounted on a gantry structure where the nozzle together with a part of the beam line can rotate around the rotation axis of the gantry.

In the context of the present invention, it is to be understood that supine position means lying horizontally with the face and torso facing up, but that positions where the legs or knees or arms are displaced with respect to a supine torso are also considered as supine. Similarly, standing and seated positions include positions where the patient is tilted forwards or backwards.

### Short description of the drawings

These and further aspects of the invention will be explained in greater detail by way of example and with reference to the accompanying drawings in which:
Fig.1a and fig. 1b represents schematically a side view and a top view respectively of a medical device according to the invention wherein the nozzle is positioned for directing a beam substantially in the direction of the axis of the MRI, the axis of the MRI being horizontal;
Fig.2 represents schematically a top view of a medical device according to the invention wherein the nozzle is positioned for directing a beam in a direction substantially perpendicular to the direction of the axis of the MRI, the axis of the MRI being horizontal;
Fig.3 represents schematically a side view of a medical device according to the invention wherein the nozzle is positioned for directing a beam substantially perpendicularly to the direction of the axis of the MRI, the axis of the MRI being vertical;
Fig.4 represents schematically a side view of a medical device according to the invention wherein the nozzle is positioned for directing a beam in a direction substantially perpendicular to the direction of the axis of the MRI, the axis of the MRI being horizontal, the beam being inclined with respect to a horizontal plane;
Fig.5 represents schematically a side view of a nozzle for use in a preferred embodiment of the invention;
Fig.6 is a data flows diagram representing the data flows in method of the invention, and
Fig.7 is a perspective view of an apparatus of the invention.

The drawings of the figures are neither drawn to scale nor proportioned. Generally, identical components are denoted by the same reference numerals in the figures.

### Detailed description of embodiments of the invention

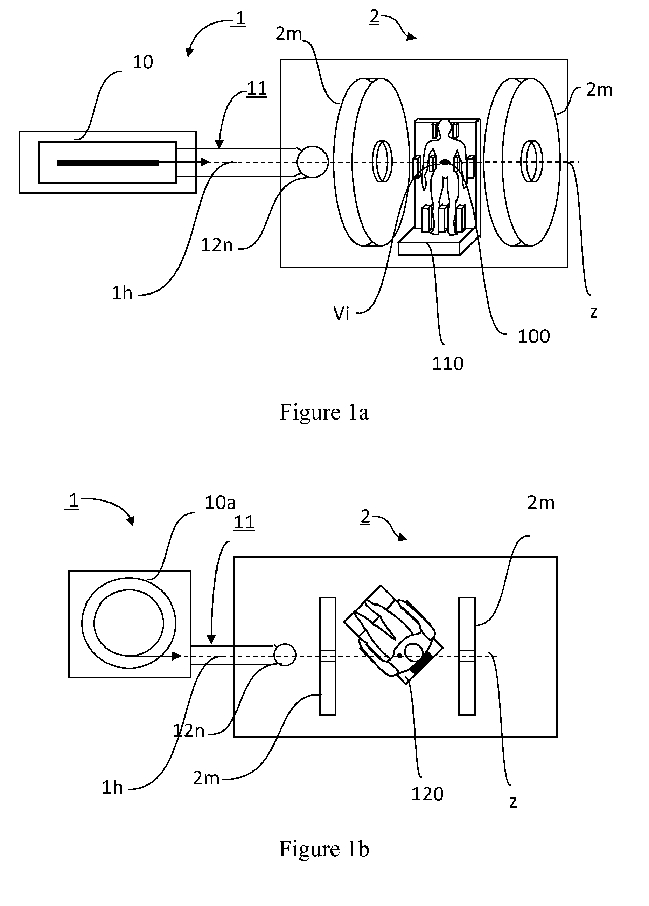

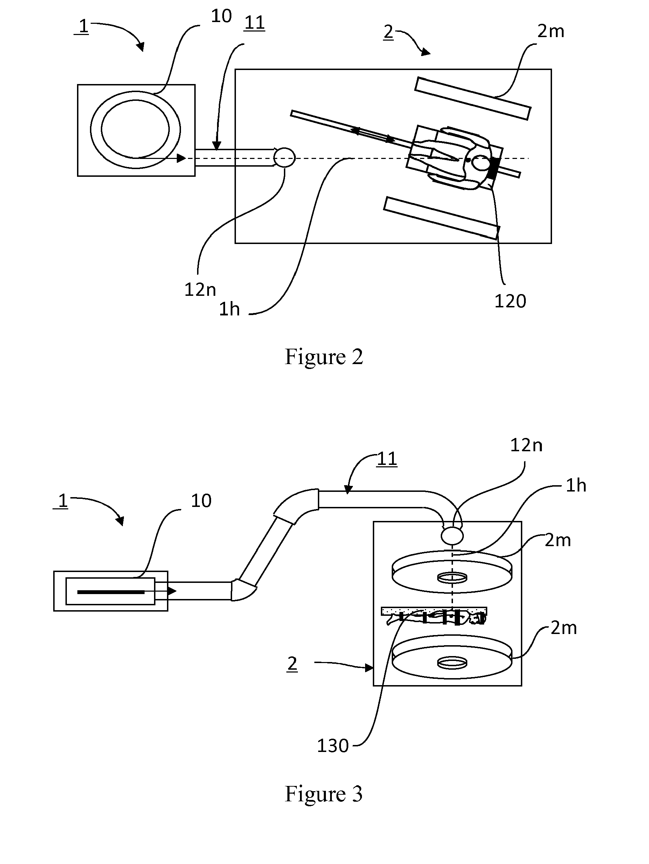

Fig.1a represents schematically a side view of a medical device according to the invention. The apparatus comprises a hadron therapy device 1 comprising hadron source 10. The hadron source 10 may comprise an accelerator 10a. Suitable accelerators include for example, a cyclotron, a synchro-cyclotron, a synchrotron, or a laser accelerator. The energy of the particles of the hadron beam 1 h when extracted from the accelerator may typically be comprised between 60 and 400 MeV, preferably between 210 and 250 MeV for proton beam, and up to 400 Mev/u for a carbon beam. A beam transport line 11 leads the hadron beam 1 h from the accelerator to a nozzle 12n. The beam transport line is preferably under vacuum. The nozzle 12n performs the functions of shaping and/or directing the beam according to the precise needs of the treatment plan. For example the nozzle may comprise scanning magnets for directing the beam to a sequence of target spots inside the target volume 40. When no scanning is performed, the beam path is along a neutral beam path. When scanning is applied, for reaching a spot away from the center 100, the beam path deviates slightly from the neutral path. The nozzle may also comprise tools for quality assurance, such as means for measuring the energy or intensity of the beam. The nozzle may be prolonged by a beam transport line arriving as near as possible to the patient, in order that the path of the beam not under vacuum is minimized. The apparatus also comprises a magnetic resonance imaging device (MRI) represented schematically by box 2 and comprises a main magnet 2m for producing the main magnetic field Bo of the MRI along the axis z of the MRI. Other components, not represented, but well known in the art, may comprise RF-excitation coils, gradient coils in the three directions X, Y, Z, antennas. The MRI 2 is designed for having a region of space wherein the magnetic field Bo produced by the main magnet 2m meets requirements regarding intensity and direction for allowing acquisition of quality MRI images. This region of space is the MRI imaging volume Vi.
In the example of Fig.1, the path of the hadron beam 1 h is represented collinear with the axis z of the MRI. However, embodiments of the invention may deviate from this collinearity have a beam path 1 h forming an angle up to 20° with the axis z of the MRI. This angular deviation may be obtained by rotating the MRI 2. In a preferred embodiment of the invention, the hadron therapy device 1 may be located at a distance of the MRI 2. The distance may be 2 m or larger, measured from the center 100 of the imaging volume Vi to the exit of the nozzle 12m. This feature has many advantages: (i) the hadron therapy apparatus 1 and the MRI 2 being further apart, the influence of the stray field of the hadron therapy apparatus on the MRI and reciprocally is minimized, (ii) in the embodiments of Fig.1, and Fig.3, where the neutral beam path 1h is parallel to the axis of the MRI, the deviated beam path deviates only from a reduced angle from the neutral beam path. Therefore, the influence of the main filed Bo is reduced with respect to a situation where the distance would be smaller and the deviation angles larger (iii) The source-axis distance (SAD) i.e. the distance between a virtual source located in the scanning magnets, and the center 100 is larger, and therefore the strength of the scanning magnets is reduced, and the scanning magnets may have smaller apertures.
The patient support 110 represented in Fig. 1 is a patient support for a standing patient. The support 110 may comprise a rotation mechanism for rotating the patient around a vertical axis. Supports known in the art may be provided for immobilizing the patient in a position. Other types of patient support may be used also in this device.

Fig.1b represents schematically a top view of the medical device 1 of Fig. 1 a. However the patient support is a seat 120. The seat comprises means for rotating the seat around a vertical axis, and is represented making an angle of about 45° with respect to the z axis of the MRI.

Fig.2 represents schematically a top view of a medical device 1 wherein the nozzle 12n is positioned for directing a beam in a direction substantially perpendicular to the direction of the axis of the MRI, the axis z of the MRI being horizontal. In the example shown, there is an angle α between the hadron beam and a perpendicular to the axis z of the MRI. This option gives more flexibility in planning treatments. The variation of the angle may be obtained by mounting the MRI 2 on a rotating platform.
The patient support 120 represented in Fig. 2 is a patient support for a seated patient. The support 120 may comprise a rotation mechanism for rotating the patient around a vertical axis and or a horizontal axis, for tilting the patient backwards or forwards. The support may be a chair as represented, or an ergonomic kneeling chair. The advantage of using an ergonomic kneeling chair is that less space is used in the narrow opening of the MRI.

Fig.3 represents schematically a side view of a medical device according to the invention wherein the nozzle is positioned for directing a beam substantially in the direction of the axis z of the MRI, the axis of the MRI being vertical. In the example show, the beam is led from the hadron source 10 to the nozzle 12n through a beam transport line 11 comprising a number of bending magnets. In an alternative, the hadron source 10 may be located above or below the MRI 2 and produce a beam in a vertical direction.
The patient support 130 represented in Fig. 3 is a patient support for a patient in prone position. The support 130 may comprise a rotation mechanism for rotating the patient around a horizontal axis.

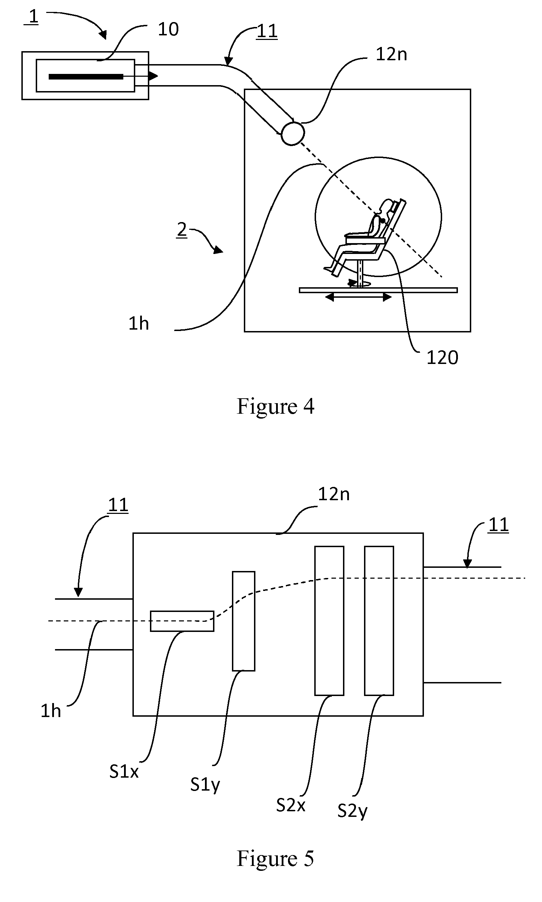

Fig.4 represents schematically a side view of a medical apparatus of the invention wherein the nozzle is positioned for directing a beam in a direction substantially perpendicular to the direction of the axis of the MRI, the axis of the MRI being horizontal, the beam being inclined with respect to a horizontal plane. The inclination angle may be any value such as 45° shown, or 60°, 30°, or same angles from below.

Fig.5 represents schematically a side view of a nozzle for use in a preferred embodiment of the invention. This nozzle is designed for the spot scanning beam delivery, in the parallel scanning mode, known from Pedroni et al., "The PSI Gantry 2 : a second generation proton scanning gantry", Z. Med. Phys. 14 (2004) 25-34. The nozzle comprises a first pair of scanning magnets 11x, S1y, for deviating the beam path from the neutral line in the directions x and y, and a second pair of scanning magnets for redirecting the beam parallel to the neutral line. Besides the known advantages of such parallel scanning, the use of such scanning in the embodiments of Fig. 1 and Fig. 3 is in the fact that the hadron beam 1 n remains parallel to the Bo field, and therefore no deviation of the hadron beam by the Bo field occurs. The part of the beam transport line at the left of fig. 5 transports the beam from the hadron source to the nozzle 12n. In all embodiments of the invention, an additional part of beam transport line 11 may be foreseen between the exit of the nozzle and the patient, thereby keeping the part of the path not under vacuum as short as possible.

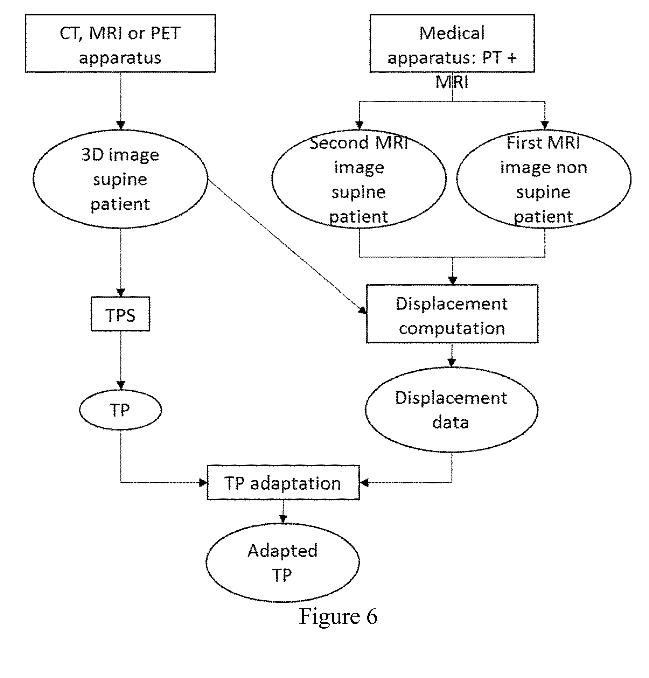

Fig.6 is a data flow diagram representing the data flow in methods of the invention. The rectangular boxes represent apparatuses or computers performing operations according to some software, and the ovals represent the data sets flowing between the boxes. The left-hand branch of the diagram represents the traditional establishment of a treatment plan: A CT scanner, MRI of PET imaging device acquires a 3D image of a patient, the patient lying in supine position. The Treatment Planning System (TPS) may be a Raystation (Research), a Pinnacle (Philips), a Xio (Elekta) or others, and provides a treatment plan TP. The right-hand branch of the diagram represents the operations performed before the treatment, in order to adapt the treatment plan TP in the knowledge of actual displacement of organs and the target volume when the patient is in the non-supine position wherein the treatment will be performed. The patient being positioned in the medical apparatus (PT+MRI), and positioned in the non-supine position wherein the treatment will be performed. A first MRI image is acquired.
In one embodiment of the invention, this image is compared with the 3D image acquired in the left-hand part of the diagram, and compared by a computer performing a displacement computation for obtaining displacement data representing the displacement of the organs of the patient and of the target volume. These displacement data are provided to a computer, together with the treatment plan TP, for performing an adaption of the treatment plan and providing and adapted treatment plan.
In another embodiment of the invention, a second MRI image is acquired, the patient being in supine position, i.e. the position wherein the 3D image was acquired. The displacement computer computes displacement data from the comparison of said first and second MRI images. The second MRI image may be acquired with the MRI of the apparatus, or in another MRI. These displacement data are again provided to a computer, together with the treatment plan TP, for performing an adaption of the treatment plan and providing and adapted treatment plan.

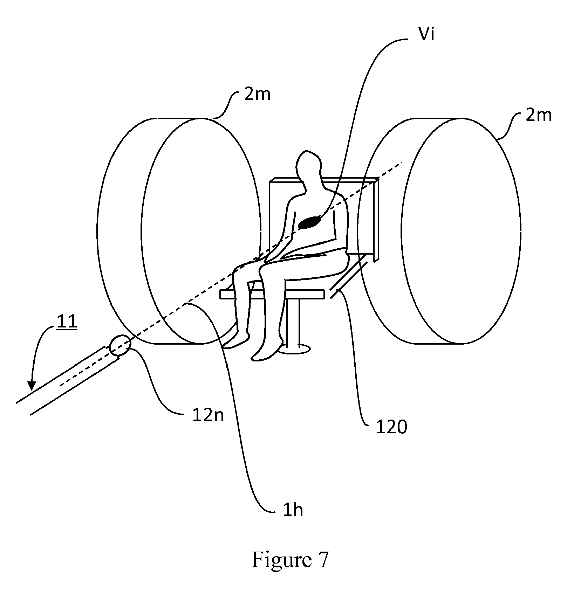

Fig. 7 is a perspective view of an apparatus of the invention having a seated patient support 120. The MRI 2 is an open MRI. An example of an open MRI is the MRopen apparatus obtainable from Paramed Medical srl, and described in US patent US7944208. This apparatus may be modified by providing an aperture or window for letting the beam pass through, along the z axis, for the embodiments of Fig. 1a, Fig. 1b and Fig. 3.

The MRI used in the invention, and the examples represented at Fig. 1 to 5 and 7 comprise two coils at a distance, providing a space between these two coils. These devices are generally known as "open MRIs". Such MRIs have the advantage that the patient is not confined in a narrow bore where he might suffer from claustrophobia. But this aspect also allows the embodiments of Fig. 2, 4 and 7, where the beam reached the patient through this opening. Also, the fact that the patient is siting, standing or lying in a more open space allow the rotation of the patient support for directing the beam under a choice of angles to the patient.

Using the device and methods of the invention, it is possible to treat patients using a hadron therapy device without a gantry. Thereby the cost of the apparatus is substantially reduced, and the space required for installing the device is reduced. It is possible to adapt a treatment plan to movements of organs resulting from the displacement of a patient from a supine position wherein treatment plan imaging was performed to a non-supine position for performing the treatment. The availability of the MRI in the hadron therapy apparatus opens the possibility of treatment in non-supine position, because the treatment plan can be adapted as needed.

## Claims

1. A medical apparatus comprising:
a) a hadron therapy device comprising a hadron source comprising a nozzle, adapted for directing a hadron treatment beam along a beam path to a target volume;
b) a magnetic resonance imaging device (MRI);
**characterised in that**,
• the MRI comprises two coils arranged at a distance and configured for generating a magnetic field in a direction along an axis in an MRI imaging volume between said two coils and around said axis, for acquiring magnetic resonance data in said MRI imaging volume, said MRI imaging volume encompassing said target volume, said imaging volume having a centre;
• said nozzle is fixed and positioned for directing a beam along a beam path substantially along said axis, the angle between said axis and said beam path being smaller or equal to 20°, or substantially perpendicularly to said axis, the angle between a perpendicular to said axis and said beam path being smaller or equal to 20°;
• it comprises a patient support system adapted for supporting a patient in a non-supine position.

2. The hadron therapy apparatus according to claim 1 **characterised in that** said patient support system is adapted for supporting a patient in a seated position.

3. The hadron therapy apparatus according to claim 1 **characterised in that** said patient support system is adapted for supporting a patient in a standing position.

4. The hadron therapy apparatus according to any of preceding claims **characterised in that** said patient support system is adapted for being rotated around a vertical axis.

5. The hadron therapy apparatus according to claim 1 **characterised in that** said patient support system is adapted for supporting a patient in a prone position.

6. The hadron therapy apparatus according to anyone of preceding claims **characterised in that** said patient support system is adapted for being rotated around a horizontal axis, said axis being a longitudinal axis of said patient support.

7. The hadron therapy apparatus according to anyone of preceding claims **characterised in that** it comprises a controller adapted for instructing said apparatus to perform the method of any of claims 10 to 14.

8. The hadron therapy apparatus according to anyone of preceding claims **characterized in that** said nozzle is positioned at a distance larger than 2m from said centre of said MRI imaging volume.

9. The hadron therapy apparatus according to anyone of preceding claims **characterized in that** said nozzle is positioned for directing a beam in a first direction along a beam path and comprises a first pair of magnets S1x, S1y configured for steering the beam in two orthogonal directions both perpendicular to said first direction, and a second pair of steering magnets S2x, S2y configured for steering the beam in a direction parallel to said first direction.

10. Method for preparing a treatment plan for treating a target volume of a patient using the apparatus of any of claims 1 to 9 comprising the steps of:
• obtaining a 3D image of a region of said patient, said region encompassing said target volume, using one of the following imaging means: a CT-scan, an MRI, a PET imaging system, said patient being in a supine positon;
• determining a treatment plan based on said 3D image;
• determining a displacement of said target volume within said patient resulting from said patient being moved from said supine to said non-supine position;
• adapting said treatment plan to said displacement;
• positioning said patient in said apparatus said patient being in said non-supine position.

11. Method according to claim 10 **characterized in that** said step of determining a displacement comprises the steps of:
• obtaining a first MRI image from said MRI, the patient being in said non-supine position;
• obtaining a second MRI image from said MRI, the patient being in a supine position;
• determining said displacement of said target volume from the comparison of said first and second MRI image.

12. Method according to claim 10 **characterized in that** said step of determining a displacement comprises the steps of:
• obtaining a first MRI image from said MRI, the patient being in said non-supine position;
• determining said displacement of said target volume from the comparison of said first MRI image and said 3D image.

13. Method for preparing a treatment plan for treating a patient using the apparatus of any of claims 1 to 9 comprising the steps of:
• obtaining a 3D image of a target volume of said patient using one of the following imaging means: a CT-scan, an MRI, a PET imaging system, said patient being in a non-supine positon;
• determining a treatment plan based on said 3D image;
• positioning said patient in said apparatus said patient being in said non-supine position.

14. Method for treating a patient using the apparatus of any of claims 1 to 9 wherein said treatment plan comprises a plurality of spots each having a spot position in said target volume, comprising the steps of:
1) performing the method of any of claims 10 to 13;
2) irradiating one or more spots of said plurality of spots
3) acquiring an image of said MRI imaging volume using said MRI;
4) comparing said image with said 3D image and adapting said treatment plan according to the differences;
5) repeating steps 2) to 4) until all spots of said plurality have been irradiated.
